Europäisches Patentamt

European Patent Office

Office européen des brevets

(19)

(11) Veröffentlichungsnummer : **0 370 994 B1**

(12) **EUROPÄISCHE PATENTSCHRIFT**

(45) Veröffentlichungstag der Patentschrift :
23.12.92 Patentblatt 92/52

(51) Int. Cl.⁵ : **A61K 31/195,** A61K 31/13,
**A61K 31/155**

(21) Anmeldenummer : 89890116.0

(22) Anmeldetag : 20.04.89

(54) **Behandlung von durch Glucose ausgelöster Quervernetzung von Collagen bei Diabetes mellitus-Patienten durch Arginin, Spermidin, Kreatin oder Agmatin.**

(30) Priorität : 25.11.88 AT 2903/88
06.03.89 AT 498/89

(43) Veröffentlichungstag der Anmeldung :
30.05.90 Patentblatt 90/22

(45) Bekanntmachung des Hinweises auf die
Patenterteilung :
23.12.92 Patentblatt 92/52

(84) Benannte Vertragsstaaten :
AT BE CH DE ES FR GB GR IT LI LU NL SE

(56) Entgegenhaltungen :
FR-A- 2 368 959
JOURNAL OF SUPRAMOLECULAR STRUC-
TURE, Band 11, 1979, Seiten 227-235, Alan R.
Liss, Inc., D.F. MOSHER et al.: "Inhibition of
blood coagulation factor XIIIa-mediated
cross-linking between fibronectin and collagen y polyamines"

(56) Entgegenhaltungen :
SCIENCE, Band 232, 27. Juni 1986, Seiten
1629-1632; M. BROWNLEE et al.:
"Aminoaguanidine prevents diabetes-induced arterialwall protein cross-linking"
PROC. NATL. ACAD. SCI. USA, Band 81, Januar 1984, Seiten 583-587; V.M. MONNIER et
al.: "Accelerated age-related browning of human collagen in diabetes mellitus"
The Merck Index, 10th Ed., 1983, p. 1251, 368,
169

(73) Patentinhaber : Lubec, Gert, Prof. Dr.
Brodschekhof 14
A-1220 Wien (AT)

(72) Erfinder : Lubec, Gert, Prof. Dr.
Brodschekhof 14
A-1220 Wien (AT)

(74) Vertreter : Beer, Manfred, Dipl.-Ing. et al
Lindengasse 8
A-1070 Wien (AT)

EP 0 370 994 B1

**Beschreibung**

Die Erfindung betrifft die Verwendung von bestimmten Wirkstoffen zur Herstellung eines Arzneimittels zur Hemmung von durch Glucose ausgelöster, pathologischer Quervernetzung von Collagen bei Diabetes mellitus-Patienten.

Die Quervernetzung von langlebigen Proteinen, wie Collagen, wird im diabetischen Zustand beschleunigt und verändert (S.L. Schnider und R.R. Kohn, J.Clin.Invest.66, 79 (1980), 67, 1630 (1981). Diese Erkenntis bildete die Grundlage für intensive Untersuchungen nach einer nicht enzymatischen Glycosylierung dieser Proteine. Neuere Arbeiten nehmen an, daß fortgeschrittene, nicht enzymatische Glycosylierungsprodukte die von Glucose abgeleiteten Quervernetzungen des Collagens bilden (vgl. V. Monnier, Cerami, A., Science 211, 491 (1981) und M.J.C. Kent, N.D. Light, A.J. Bailey, Biochemical J. 225, 745 (1985)). Ein Reaktionsmechanismus für diese abnormale Quervernetzung ist im beigefügten Reaktionsschema (Mitte) wiedergegeben.

Glucose reagiert mit Aminogruppen von Proteinen in einer reversiblen, nukleophilen Additionsreaktion zu einem Schiff'sche Basen-Addukt (Aldimin), das sich dann in einer Amadori-Umlagerung in das stabilere, aber dennoch reaktionsfähige Amadori-Produkt umwandelt (vgl. H.B. Mortensen und C. Christophersen, Clin.Chim.Acta 134, 317 (1983)).

Das so erhaltene Amadori-Produkt durchläuft dann eine Reihe weiterer langsamer Reaktionen mit Aminogruppen anderer Proteine, um von Glucose abgeleitete, intermolekulare Quervernetzungen zu bilden, wie das unlängst beschriebene, fortgeschrittene Glycosylierungsprodukt, nämlich 2-(2-Furoyl)-4(5)-(2-furanyl)-1H-imidazol (S. Pongor, P.C. Ulrich. F.A. Bencsath, A. Cerami, Proc. Natl.Acad. Sci USA, 81, 2684 (1984)).

Fortgeschrittene Glycosylierungsprodukte häufen sich über längere Zeiträume fortlaufend auf langlebigen Proteinen, wie Collagen an (M. Brownlee, H. Vlassara, A. Cerami in: Diabetes Complications Scientifique and Clinical Aspects. M.J.C. Grabbe, Ed. (Pitman, London 1986)).

In Journal of Supramolecular Structure, Band 11, 1979, Seiten 227-235, Alan R. Liss, Inc; D.F. Mosher et al.: "Inhibition of blood coagulation factor XIII$_a$-mediated cross-linking between fibronectin and collagen by polyamines" wird beschrieben, daß Spermidin geeignet sei, die durch den Blutgerinnungsfaktor XIII$_a$ vermittelte Quervernetzung von Fibronectin und Collagen zu trennen.

Im Collagen von diabetischen Personen wird diese altersbedingte Anhäufung von fortgeschrittenen Glycosylierungsprodukten durch einen über längere Zeit erhöhten Glucosespiegel beschleunigt (Monnier, V., Kohn, R.R. Cerami, A., Proc. Natl. Acad. Sci USA 81, 583 (1984)). Ausgehend von der Überlegung, daß die durch Glucose induzierte Bildung von Proteinquervernetzungen verhindert werden könnte, wenn die reaktiven Carbonyle der anfänglichen Glycosylierungsprodukte (Ketoamine) pharmakologisch blockiert werden können, untersuchten Brownlee und Mitarbeiter den Einfluß einer nukleophilen Hydrazinverbindung (Aminoguanidin, $H_2N-C(=NH)-NH-NH_2$) auf diesen Reaktionsablauf (Brownlee, M., H. Vlassara, A.Kooney, P. Ulrich, A. Cerasmi Science 232, 1629 (1986)). Sie zeigten, daß Aminoguanidin in vitro die Bildung fortgeschrittener Glycosylierungsprodukte und die durch Glucose induzierte Collagenquervernetzung verhindert (Reaktionsschema, links). Ihre Ergebnisse zeigten auch, daß an Ratten verabreichtes Aminoguanidin die durch Diabetes induzierte Anhäufung von fortgeschrittenen Glycosylierungsprodukten und eine abnormale Quervernetzung von Protein im Bindegewebe von Arterienwänden verhindert. Der Einfluß von Aminoguanidin auf die Bildung fortgeschrittener Glycosylierungsprodukte wurde durch Messen der spezifischen Fluoreszenz, wie sie früher für Collagen beschrieben worden ist, beurteilt.

Betreffend die Albuminglycosylierung wurde festgestellt, daß durch Aminoguanidin die Bildung eines fortgeschrittenen Albuminglycosylierungsproduktes verhindert wird, wogegen die Amadori-Umwandlung im wesentlichen unverändert stattfindet.

Die Wirkung von Aminoguanidin auf die Collagenquervernetzung in vitro wurde durch Natriumdodecylsulfat- Polyacrylamidgelelectrophorese (SDS-PAGE) von Cyanbromidspaltprodukten nativer Collagenfibrillen bestimmt. Mit der Zeit zeigte das Gelmuster von Cyanbromidspaltprodukten aus Collagen, das mit Glucose inkubiert wurde, erhöhte Werte an quervernetzten Peptiden mit hohem Molekulargewicht. Die Anwesenheit von Aminoguanidin in der Inkubationsmischung verringerte die Menge an quervernetzten Peptiden mit hohem Molekulargewicht signifikant.

Die in vivo-Wirkung von Aminoguanidin wurde an nichtdiabetischen und alloxandiabetischen Ratten gezeigt. Es wurden täglich intraperitoneal Injektionen von Aminoguanidin verabreicht. Es wurde die Menge an entstandenen, fluoreszierenden, fortgeschrittenen, nicht enzymatischen Glycosylierungsprodukten, bestimmt. Der Grad der Quervernetzung des Aorta-Bindegewebes war in Aminoguanidin-Tieren kleiner als bei solchen, die kein Aminoguanidin erhielten.

Die Löslichkeit von Collagen, ein anderes Kennzeichen für die Quervernetzung, ging bei mit Aminoguanidin behandelten Tieren auf normale Werte zurück.

So wie die meisten Hydrazine, besitzt auch Aminoguanidin eine außerordentlich hohe Toxizität. Es ist somit in der Praxis nicht einsetzbar.

Die Anwendung von Arginin in der Medizin ist schon bekannt, nämlich bei der Behandlung von Arginino-succinasemangel, Asthenospermie und Immunomodulation.

Spermidin, Kreatin und Agmatin sind auch Substanzen, deren biochemische Eigenschaften schon beschrieben worden sind. (Siehe THE MERCK INDEX, tenth edition, 1983, S. 1251, S. 368, S. 169).

Weiterhin wird im JOURNAL OF SUPRAMOLECULAR STRUCTURE, Band 11, S. 227, R. Liss, berichtet, daß Spermidin die Quervernetzung zwischen Fibronektin und Collagen I hemmt.

Aufgabe der Erfindung ist es, Substanzen anzugeben, welche die Bildung von Quervernetzungen in Collagenproteinen in einer mit Aminoguanidin vergleichbaren Wirkungsweise verhindern und die nicht toxisch sind.

Die Erfindung schlägt die Verwendung von Arginin, insbesondere L(+)-Arginin (HCOOC-CH(NH$_2$)-(CH$_2$)$_3$-NH-C(NH)-NH$_2$), Agmatin, dem Decarboxylierungsprodukt von Arginin (CH$_2$(NH$_2$)-(CH$_2$)$_3$-NH-C(NH)-NH$_2$), Kreatin (N-Amidinosarkosin, H$_2$N-C(NH)-N(CH$_3$)-CH$_2$-COOH) oder Spermidin (N-(3-Aminopropyl)-1.4-butandiamin, H$_2$N-(CH$_2$)$_3$-N-H-(CH$_2$)$_4$-NH$_2$) oder einem Salz derselben zur Herstellung eines Arzneimittels zur Hemmung pathologischer Quervernetzung in Collagen bei Diabetes- mellitus-Patienten vor. An Stelle von Arginin kann auch ein Analogon von Arginin, nämlich Canavanin (HN(NH$_2$)C-NH-O-CH$_2$-CH$_2$-CH(NH$_2$)-COOH verwendet werden.

Arginin ist eine Aminosäure, die nicht toxisch ist und die in allen Proteinen in der täglichen Nahrung enthalten ist. Spermidin und Kreatin kommen im menschlichen Organismus in signifikanten Mengen vor.

Für die nachstehend beschriebenen Untersuchungen wurde die freie Base von L-Arginin verwendet.

Es wurden folgende in vitro Untersuchungen ausgeführt:

1. Fluoreszenztest nach dem Verfahren von Monnier V. et.al. (s. oben)

2. SDS-PAGE von mit Glucose inkubierten, isolierten Collagenen mit und ohne L-Arginin, wobei Aminoguanidin als Vergleichssubstanz herangezogen wurde.

3. Inkubationsversuche mit verschiedenen Collagenpräparaten, interstitiellen Collagenen und Basalmembrancollagen.

Es wurden folgende in vivo-Experimente ausgeführt, wobei sowohl Schweizer Mäuse als auch spontan diabetische KK-Mäuse verwendet wurden:

4. Löslichkeitsversuche

5. SDS-PAGE von isolierten Collagenen

6. Bestimmung des Nierengewichtes durch Autopsie (Serumparameter: Fructosamin)

7. Nichtenzymatische Glycosylierungstestreihen und

8. Fluoreszenztestreihen.

Wie bereits erwähnt, wurden für die in vivo-Versuche zwei verschiedene Versuchstierarten, nämlich Schweizer Mäuse und spontan diabetische KK-Mäuse verwendet.

Die täglich oral verabreichte Dosis betrug 50 mg/kg Körpergewicht über einen Zeitraum von 5 Wochen.

Nebenwirkungen wurden weder klinisch noch bei einer durchgeführten Autopsie festgestellt.

Dies stimmt mit klinischen Berichten von Wissenschaftern überein, die Arginin für andere Zwecke, z.B. für Diätergänzung im gesunden Zustand, und für Krankheiten, wie Argininosuccinasemangel, Asthenospermie und Immunomodulation untersucht hatten.

Überdies ist Arginin im Handel, z.B. in Apotheken, rezeptfrei erhältlich und wurde in den USA durch die FDA bereits als Diätzusatzmittel zugelassen.

In vitro Versuche:

1. Fluoreszenzversuchsreihe von in vitro nicht enzymatisch glycosyliertem Collagen Type I (interstitielles Collagen) und Type III.

Collagen in seinem nativen Zustand wurde in 200 mM Glucose sowohl in Gegenwart als auch in Abwesenheit von Arginin bzw. Aminoguanidin in equimolaren Verhältnissen nach dem Verfahren von Brownlee (Brownlee, M., H.Vlassara, A. Kooney, P. Ulrich, A. Cerami Science 232, 1629 (1986)) inkubiert. Collagene ohne Glucose im Inkubationsmedium wurden als Vergleichsproben verwendet. Eine signifikante Hemmung der Fluoreszenz gemäß der Methodik von Monnier und Mitarbeitern (Monnier, V., Kohn, R.R., Cerami, A., Proc.Natl.Acad. Sci USA 81, 583 (1984)) wurde für Aminoguanidin und Arginin bei allen Collagenarten beobachtet.

2. Eine SDS-PAGE gemäß dem Verfahren von Brownlee mit der Abänderung, daß die Collagenspaltprodukte durch Collagenasedigestion statt durch Bromcyanspaltung erhalten wurden, zeigte höher molekulargewichtige Collagenspaltprodukte in den Proben ohne Aminoguanidin bzw. Arginin. Bei den Proben, die Arginin bzw. Aminoguanidin enthielten, wurde ein Molekulargewichts-Muster ähnlich dem von nichtglycosyliertem Collagen beobachtet.

3. Ein analoger Versuch mit Basalmembrancollagen der Type IV zeigte die Aktivität von Arginin gegenüber diesem vaskulären Collagensystem auf.

In vivo Versuche:

Es wurden normale Schweizer Mäuse, 10 Stück je Gruppe, weiß, weiblich, 1 Jahr alt, 6 Wochen lang mit Arginin behandelt, wobei je Tag 50 mg/kg Körpergewicht Arginin oral in Leitungswasser verabreicht wurden. Die Versuchstiere hatten freien Zugang zu fester Nahrung, nämlich Ratten-Futter. Zehn entsprechende Tiere dienten als Vergleichstiere.

4. Löslichkeitsversuch: Collagen wurde aus der Haut, der Leber und der Niere der Versuchstiere extrahiert. Zwischen behandelten und unbehandelten Versuchstieren zeigte sich kein Unterschied in der Extrahierbarkeit von Collagen.

5. Bei einer SDS-PAGE wurden keine Unterschiede zwischen den extrahierten Collagenen beider Gruppen von Versuchstieren aufgezeigt, was zeigt, daß keine Beeinflussung der normalen Quervernetzung vorliegt.

6. Bei einer Autopsie wurden keine Unterschiede zwischen den beiden Gruppen von Versuchstieren festgestellt. Es wurden weder Unterschiede in den Gewichten von Organen noch pathologische Feststellungen aufgezeigt.

7. Es bestand ein signifikanter Unterschied bei der nichtenzymatischen Glycosylierung von Serumprotein, wie sich durch einen Fructosamin-Versuch ausdrücken ließ. Die mit Arginin behandelte Gruppe zeigte niedrige Gehalte an nichtenzymatischen Glycosylierungen des Serumproteins. Dies zeigt eine Wirkung auf die nichtenzymatische Glycosylierung, die bis zu einem gewissen Ausmaß auch unter physiologischen Bedingungen stattfindet.

8. Die Fluoreszenz von aus der Haut, der Leber und der Niere nach der oben beschriebenen Methode extrahiertem Collagen zeigte signifikante Unterschiede, die den positiven Einfluß auf die nichtenzymatische Glycosylierung, nämlich eine Verminderung dieses Prozesses, aufzeigte.

10 Monate alte KK-Mäuse, weiß, weiblich, fettleibig und diabetisch wurden 5 Wochen lang mit Arginin behandelt. Es wurde die gleiche Dosierung wie oben angegeben verwendet. Die 10 KK-Mäuse dienten als Vergleichstiere, wobei das Protokoll wie für die Schweizer Mäuse erstellt wurde.

9. Löslichkeitsversuch: Collagen wurde aus der Haut, der Leber und der Niere der Versuchstiere extrahiert. Es gab einen signifikanten Unterschied, da die Löslichkeit bei den behandelten Tieren erheblich höher war als bei den nichtbehandelten Vergleichstieren, was einen positiven pharmakologischen Effekt von Arginin anzeigt, da die geringe Löslichkeit im diabetischen Zustand einer der pathogenen Faktoren für Langzeitkomplikationen bei dieser Krankheit ist.

10. Bei SDS-PAGE wurden signifikante Unterschiede aufgezeigt. Nichtbehandelte KK-Mäuse zeigten Collagenspaltprodukte mit höherem Molekulargewicht als behandelte KK-Mäuse. Dies zeigt die positive Wirkung von Arginin auf die Quervernetzung und die nichtenzymatische Glycosylierung auf.

11. Bei einer Autopsie wurden keine Unterschiede im allgemeinen Erscheinungsbild aufgezeigt. Es wurden in beiden Gruppen keine makroskopischen, pathologischen Feststellungen ermittelt, jedoch wurde bei der Autopsie von diabetischen Mäusen (analog dem Menschen) festgestellt, daß nichtbehandelte Mäuse ein signifikant höheres Nierengewicht besitzen als mit Arginin behandelte Mäuse.

12. Die Serumfructosaminkonzentrationen ausgedrückt als Morpholinfructose waren bei den mit Arginin behandelten Tieren statistisch signifikant kleiner, was einen positiven pharmakologischen Effekt auf die nichtenzymatische Glycosylierung aufzeigt.

13. Fluoreszenzversuche von aus den Mäusen isolierten Collagenen zeigten signifikant geringere Werte bei den behandelten Tieren, was eine positive Wirkung auf die nichtenzymatische Glycosylierung aufzeigte.

Die oben wiedergegebenen Daten zeigten die positive, der Wirkung von Aminoguanidin ähnliche Aktivität von Arginin in vitro und in vivo, nämlich die Hemmung von fataler, glucose-induzierter Quervernetzung von Collagen im diabetischen Zustand.

Der Reaktionsmechanismus für Collagen-Protein in seiner glycosylierten Form mit Arginin ist im Reaktionsschema rechts wiedergegeben, wobei Arginin mit einer seiner reaktionsfähigen, durch ein Sternchen hervorgehobenen Stellen mit der aktiven Karbonylgruppe des Ketoamins reagieren kann. Der Reaktionsmechanismus bei Verwendung von Spermidin, Kreatin und Agmatin entspricht dem im Reaktionsschema für Arginin gezeigten Mechanismus.

An Stelle von Arginin, Spermidin, Kreatin und Agmatin kann ein pharmazeutisch annehmbares Salz derselben (z.B. Glutamat) oder ein Analogon derselben (z.B. Canavanin als Analogon von Arginin) verwendet werden.

Die an KK-Mäusen ausgeführten Untersuchungen wurden an spontan diabetischen db/db Mäusen aus Großbritannien durchgeführt. Es ergaben sich in diesem System dieselben korrelierenden Ergebnisse wie bei den KK-Mäusen. Auch dieses System ist ein geeignetes Modell für Diabetes Typ II.

Es wurden elektronenmikroskopische Untersuchungen an Nierengewebsentnahmen von Mäusen mit Diabetes mellitus, KK-Mäusen, aus der BRD durchgeführt.

Es wurden morphometrisch die Gefäss(Glomerulum-)basalmembrandicken ausgewertet und die Mesangiumproliferation gemessen, und mit L-Arginin behandelte Tiere unbehandelten Kontrolltieren gegenübergestellt.

Es ergaben sich signifikant dünnere Basalmembranen und signifikant verminderte Mesangiumkollagenproliferation im Panel der behandelten Tiere.

Fig. 1 zeigt eine elektronenmikroskopische Aufnahme eines Glomerulum ohne Argininbehandlung bei einer KK-Maus.

Fig. 2 zeigt mit derselben Vergrößerung ein Glomerulum einer mit Arginin behandelten KK-Maus.

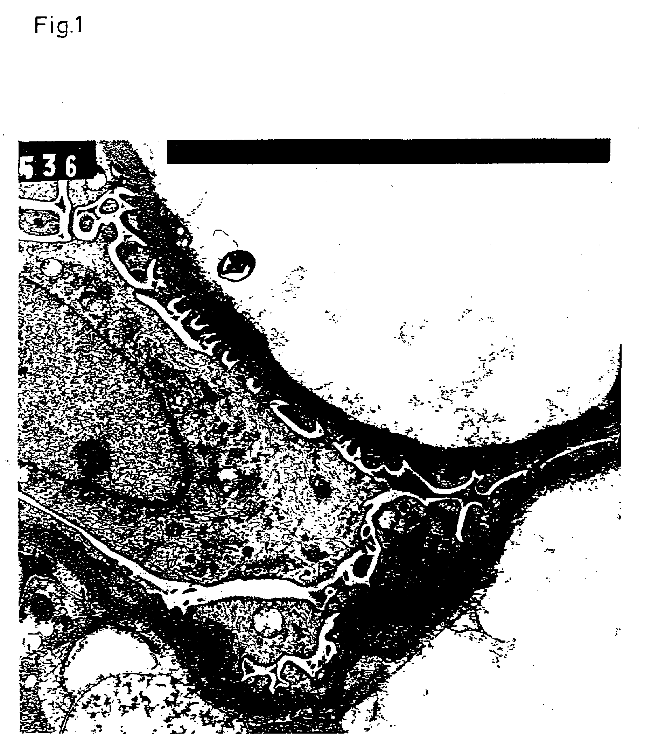

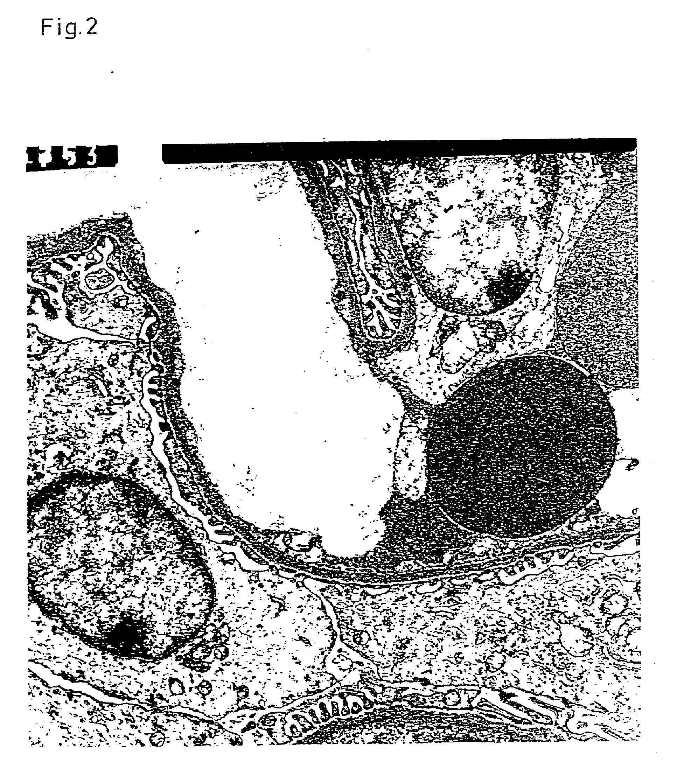

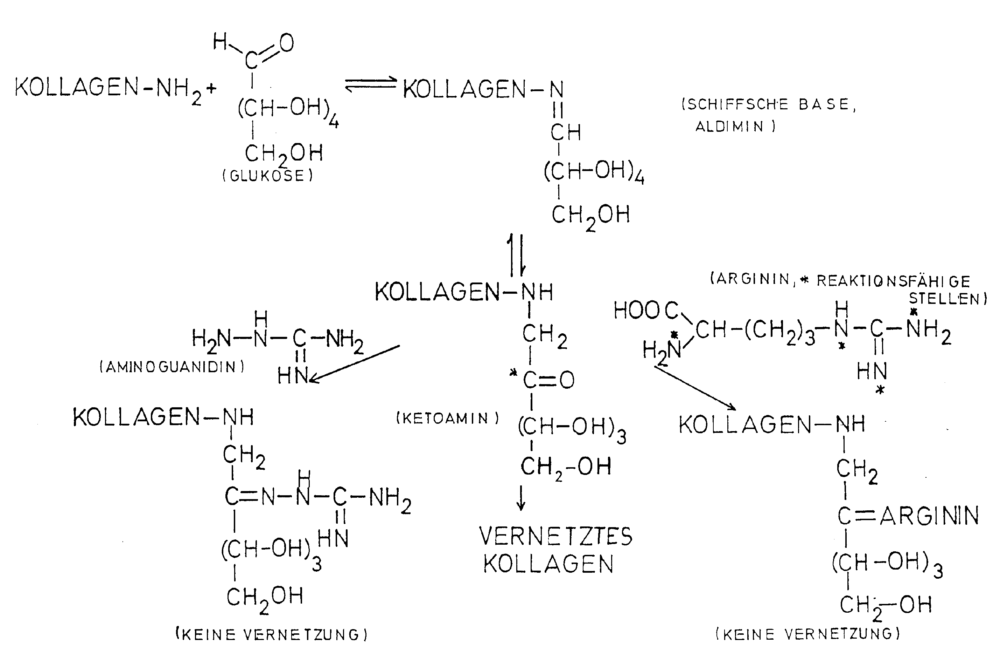

Es ist in Fig. 1 eine im Vergleich zu Fig. 2 dickere Basalmembran und geringere Proliferation im Mesangium erkennbar. In Fig. 2 ist keine Mesangiumproliferation und eine dünnere GMB(Glomerulumbasalmembran) erkennbar.

Die mit der freien Base von L-Arginin durchgeführten Versuche wurden auch mit Agmatin ausgeführt. Dabei wurden mit den in den oben beschriebenen Versuchen erzielten Ergebnissen vergleichbare Ergebnisse erzielt.

Bei entsprechenden Untersuchungen an Tieren mit durch Streptomycin induziertem Diabetes wurden analoge Ergebnisse erzielt.

Auch bei Untersuchungen an spontan diabetischen BB-Ratten wurden entsprechende Ergebnisse erzielt.

Die mit Arginin und Agmatin durchgeführten Versuche wurden auch mit Spermidin und Kreatin ausgeführt. Dabei wurden mit den weiter oben beschriebenen Versuchen vergleichbare Ergebnisse erzielt.

Die in den Versuchen verwendeten Tiermodelle repräsentieren die Behandlungsmöglichkeiten bei Diabetes Typ I und Diabetes Typ II.

Geeignete pharmazeutische Präparate zur Hemmung der Quervernetzung von Collagen werden vorteilhaft und in einfacher Weise durch Kombinieren von Arginin, Spermidin, Kreatin, Agmatin, einem Salz oder einem Analogon davon mit pharmazeutisch geeigneten, chemisch inerten Füllstoffen, Trägern, Streckmitteln und Excipients, wie sie allgemein zur Herstellung von Arzneimitteln verwendet werden, die oral oder parenteral verabreicht werden, oder die lokal injiziert werden sollen, und die in dieser Beschreibung un den Patentansprüchen allgemein und kollektiv als "Arzneimittelhilfsstoff" bezeichnet werden, hergestellt. Die erfindungsgemäßen Verbindungen können in Form von Tabletten, Pulvern, Kapseln, Suspensionen, Lösungen, Emulsionen und ähnlichen Dosierungsformen kompoundiert oder formuliert werden. Die Zubereitungen können durch Vermischen von Arginin, Spermidin, Agmatin, Kreatin, einem Analogon oder einem Salz derselben, vorzugsweise in wasserlöslicher Form, mit diesen üblichen Verdünnungsmitteln oder Tablettierzusätzen, wie Cellulosepulver, Maisstärke, Lactose, Talkum, Stearinsäure, Magnesiumstearat, Gummis oder dergleichen, nach den auf diesem Fachgebiet bekannten üblichen Herstellungsmethoden hergestellt werden.

Wenn das Produkt parenteral verabreicht oder lokal injiziert werden soll, können die erfindungsgemäßen Mittel vorzugsweise in ihrer nicht toxischen, wasserlöslichen Form mit Trägern kombiniert werden, wie Wasser, Salzlösung, Glucoselösung oder dergleichen.

Wirksame Mengen beliebiger der erfindungsgemäß verwendeten Verbindungen können dem Körper eines Diabetes-Patienten nach einer von verschiedenen Methoden verabreicht werden, beispielsweise oral, wie in Kapseln oder Tabletten, parenteral in Form von sterilen Lösungen oder Suspensionen, intravenös in steriler Lösung oder lokal in Form von sterilen Lösungen oder Suspensionen.

**Patentansprüche**

1. Verwendung von Arginin oder dessen Analogon Canavanin, Spermidin, Kreatin, Agmatin oder einem Salz derselben zur Herstellung eines Arzneimittels zur Hemmung pathologischer Quervernetzung in Collagen bei Diabetes-mellitus-Patienten.

2. Verwendung nach Anspruch 1 von L(+)-Arginin oder einem Salz desselben davon.

**Claims**

1. Use of arginine or its analogues canavinine, spermidine, creatine, agmatine or a salt thereof for producing a medicinal substance for arresting pathological cross-linking in collagen in patients with diabetes mellitus.

2. Use according to claim 1 of L(+)-arginine or a salt thereof.

**Revendications**

1. Utilisation de l'arginine, ou de ses analogues que sont la canavanine, la spermidine, la créatine et l'agmatine, ou d'un sel de ces composés, pour la préparation d'un médicament destiné à empêcher la réticulation pathologique du collagène chez les patients atteints de diabète sucré.

2. Utilisation, selon la revendication 1, de la L(+)-arginine ou de l'un de ses sels.

Fig.1

Fig.2

KOLLAGEN–NH$_2$ + 

$$\begin{array}{c} H \\ C=O \\ | \\ (CH-OH)_4 \\ | \\ CH_2OH \end{array}$$

(GLUKOSE)

$\rightleftharpoons$ 

KOLLAGEN–N

$$\begin{array}{c} \| \\ CH \\ | \\ (CH-OH)_4 \\ | \\ CH_2OH \end{array}$$

(SCHIFFSCHE BASE, ALDIMIN)

$\updownarrow$

KOLLAGEN–NH

$$\begin{array}{c} | \\ CH_2 \\ | \\ {}^*C=O \\ | \\ (CH-OH)_3 \\ | \\ CH_2-OH \end{array}$$

(KETOAMIN)

$\downarrow$

VERNETZTES KOLLAGEN

(AMINOGUANIDIN)

$$H_2N-\overset{\overset{\displaystyle H}{|}}{N}-\overset{\overset{\displaystyle NH_2}{\|}}{C}$$
HN

$\swarrow$

KOLLAGEN–NH

$$\begin{array}{c} | \\ CH_2 \\ | \\ C=N-\overset{H}{N}-C-NH_2 \\ | \quad\quad \| \\ (CH-OH)_3 \; HN \\ | \\ CH_2OH \end{array}$$

(KEINE VERNETZUNG)

(ARGININ, * REAKTIONSFÄHIGE STELLEN)

$$HOOC-\underset{H_2N^*}{\overset{}{C}}H-(CH_2)_3-\overset{*}{N}-\overset{*}{C}-NH_2$$
HN
*

$\searrow$

KOLLAGEN–NH

$$\begin{array}{c} | \\ CH_2 \\ | \\ C=ARGININ \\ | \\ (CH-OH)_3 \\ | \\ CH_2-OH \end{array}$$

(KEINE VERNETZUNG)

8